# EUROPEAN PATENT SPECIFICATION

(11) **EP 2 575 811 B1**
(45) Date of publication and mention of the grant of the patent: **12.08.2020**
(21) Application number: 11738841.3
(22) Date of filing: 02.06.2011
(51) Int. Cl.: A61P 31/04, A61K 31/424, A61K 31/546, A61K 9/20

(54) **PHARMACEUTICAL FORMULATION COMPRISING CEFPODOXIME PROXETIL AND CLAVULANIC ACID**
PHARMAZEUTISCHE FORMULIERUNG ENTHALTEND CEFPODOXIME PROXETIL UND CLAVULANSÄURE
COMPOSITION PHARMACEUTIQUE COMPRENANT CEFPODOXIME PROXETIL ET ACIDE CLAVULANIQUE

(30) Priority: 03.06.2010 TR 201004466
(43) Date of publication of application: 10.04.2013
(73) Proprietor: Bilgic, Mahmut, 34220 Esenler-Istanbul (TR)
(72) Inventor: Bilgic, Mahmut, 34220 Esenler-Istanbul (TR)
(86) International application number: PCT/TR2011/000147
(87) International publication number: WO 2011/152807

(56) References cited:
- WO-A1-96/01109
- WO-A1-02/067943
- WO-A1-2005/065685
- WO-A1-2007/086012
- LIVERMORE D M ET AL: "Orthodox and unorthodox clavulanate combinations against extended-spectrum beta-lactamase producers", CLINICAL MICROBIOLOGY AND INFECTION : THE OFFICIAL PUBLICATION OF THE EUROPEAN SOCIETY OF CLINICAL MICROBIOLOGY AND INFECTIOUS DISEASES JAN 2008 LNKD- PUBMED:18154546,, vol. 14 Suppl 1, 1 January 2008 (2008-01-01), pages 189-193, XP002637026,

## Description

The present invention relates to pharmaceutical formulations comprising cefpodoxime proxetil and clavulanic acid derivatives thereof as the active agents.

### Background of the Invention

Cefpodoxime proxetil (formula I), chemical name of which is pivaloyloxymethyl 7-[2-(2-amino-thiazole-4-yl]-2-(syn)-methoxyimino-acetamido]-3-methoxymethyl-3-cefem-4-carboxylate, was first disclosed in the patent numbered EP0049118.

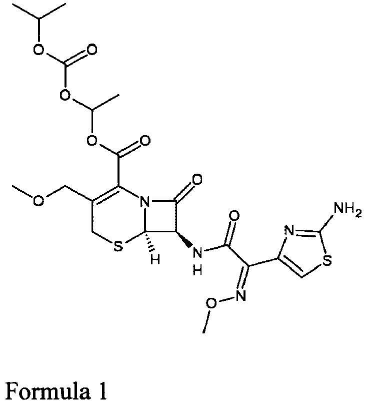

Clavulanic acid, on the other hand, is a beta-lactamase inhibitor illustrated in Formula 2.

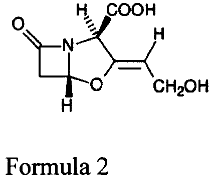

Clavulanic acid and derivatives thereof (for instance its salts such as potassium clavulanate) are known as the beta-lactamase inhibitors which withstand the beta-lactamase-originated resistance mechanism by suppressing the activity of beta-lactamase enzymes.

In the patent application numbered in WO9747301, it is disclosed that crystalline potassium clavulanate existing in the form of rod-like or needle-like crystals are agglomerated into platelike crystals or aggregated into loosely formed bundles, which leads to processing difficulties. It is also explained that the material cannot always flow readily and can be difficult to sieve due to its tendency to form agglomerates or aggregates.

In the oral dosage forms comprising cefpodoxime proxetil and clavulanic acid such as tablets, sachet or capsules, some problems have been seen during the production and quality control stages. The smooth flow of the mixture cannot be obtained due to some reasons such as irregularities in the surfaces of the particles, the possible interparticle friction and the flowability problems of potassium clavulanate material.

Accordingly, in cases where the formulations are compressed into tablets/ filled into sachets or capsules, this problem leads to significant variations in tablet/sachet or capsule weight and hence increase in the relative standard deviation. The weight uniformity can not be provided due to the observed deviations in tablet/sachet or capsule, in other words in the end product, weight and this causes some problems for the producer in the production and quality control stages.

As is seen, there is a requirement for the development of new oral pharmaceutical formulations comprising the combinations of cefpodoxime proxetil and clavulanic acid derivatives which prevent obtaining tablets/sachets or capsules having nonuniform weight of said formulation and eliminate the problems seen in the production and quality control stages.

### Description of the Invention:

The present invention relates to pharmaceutical formulations comprising cefpodoxime proxetil and clavulanic acid derivatives and the preparation methods thereof. In the present invention, pharmaceutical formulations comprising cefpodoxime proxetil and clavulanic acid derivatives wherein a combination of glidants consisting of a mineral having Mohs' hardness value in the range of 1-5 and another glidant consisting of a mineral having Mohs' hardness value in the range of 5.1-10 is used and the ratio of the first glidant to the second glidant is in the range of 5:1 to 1:5, preferably 2:1 to 1:3 and more preferably 1:1. The inventors have surprisingly found that during the preparation of said pharmaceutical formulations developed according to the present invention, a high relative standard deviation and nonuniformity of weight in the end product is eliminated owing to the fact that a smooth flow of the formulations is provided.

The first aspect of the present invention is the pharmaceutical formulation comprising cefpodoxime proxetil and clavulanic acid derivatives in combination wherein a combination of glidants consisting of a first glidant wherein said glidant consists of a mineral having Mohs' hardness value in the range of 1-5 and a second glidant wherein said glidant consists of a mineral having Mohs' hardness value in the range of 5.1-10 is used and the ratio of the first glidant to the second glidant is in the range of 5:1 to 1:5, preferably 2:1 to 1:3 and more preferably 1:1.

Cefpodoxime proxetil that can be used in the pharmaceutical formulation of the present invention can be in the form of its solvates, hydrates, enantiomers, racemates, organic salts, inorganic salts, polymorphs, crystalline and amorphous forms or free base form and/or a combination thereof.

Clavulanic acid that can be used in the pharmaceutical formulation of the present invention can be in the form of its solvates, hydrates, enantiomers, racemates, organic salts, inorganic salts, polymorphs, crystalline and amorphous forms or free base form and/or a combination thereof. Potassium clavulanate is preferably used in the present invention.

In another aspect, the end product comprising the formulation according to present invention can be in conventional tablet, film coated tablet, sachet or capsule form.

The first glidant consisting of a mineral having Mohs' hardness value in the range of 1-5 can be selected from a group comprising talc, tribasic calcium phosphate and calcium carbonate. Preferably, talc is used as the first glidant.

The second glidant consisting of a mineral having Mohs' hardness value in the range of 5.1-10 can be selected from a group comprising titanium dioxide, silicon dioxide (SiO₂) and magnesium silicate. Preferably, silicon dioxide is used as the second glidant.

Another aspect of the present invention is the pharmaceutical formulation comprising cefpodoxime proxetil and clavulanic acid derivatives in combination wherein a combination of talc and silicon dioxide is used and the ratio of talc to silicon dioxide is in the range of 5:1 to 1:5, preferably 2:1 to 1:3 and more preferably 1:1.

In the oral dosage forms comprising cefpodoxime proxetil and clavulanic acid derivatives, adhesion of the formulation to the surface of the dies and punches is also one of the important problems seen in the preparation of said formulations. The inventors have found that the amount of the glidants used in the formulation is an important parameter affecting the adhesion of the formulation.

They have observed that when each component of the glidant combination is used in amount more than % 0.6 by weight with respect to the weight of the unit dose, adhesion of the formulation comprising cefpodoxime proxetil and clavulanic acid derivatives can be eliminated.

Another aspect of the present invention is the pharmaceutical formulations comprising cefpodoxime proxetil and clavulanic acid derivatives wherein each component of the abovementioned glidant combination is used in an amount more than % 0.6 by weight with respect to the weight of the unit dose. Preferably each component of the glidant combination is used in an amount in the range of 0.6 to 3%.

The formulation of the present invention can comprise various excipients such as, but not limited to, disintegrants, lubricants, diluents, surfactants and optionally coating agents in addition to cefpodoxime proxetil, clavulanic acid and glidants.

The diluent that can be used in the pharmaceutical formulation of the present invention can be selected from, but not limited to, a group comprising calcium carbonate, calcium sulphate, dibasic calcium phosphate, tribasic calcium phosphate, calcium sulfate, microcrystalline cellulose, lactose, magnesium carbonate, magnesium oxide, maltodextrin, maltose, mannitol, sodium chloride, sorbitol, starch, xylitol or combinations thereof. Preferably, starch is used as the diluent in the pharmaceutical formulation of the present invention.

In an aspect, the pharmaceutical formulation comprising cefpodoxime proxetil and clavulanic acid derivatives in combination comprises a diluent in the range of 20% or less, preferably in the range of 2-15%, more preferably in the range of 4-8%.

The lubricant that can be used in the pharmaceutical formulation of the present invention can be selected from, but not limited to, a group comprising calcium stearate, magnesium stearate, PEG 6000, polyvinyl alcohol, potassium benzoate, talc, sodium benzoate or combinations thereof. Preferably, magnesium stearate is used as the lubricant in the pharmaceutical formulation of the present invention.

The disintegrant that can be used in the pharmaceutical formulation of the present invention can be selected from, but not limited to, a group comprising carboxymethyl cellulose calcium, carboxymethyl cellululose sodium, microcrystalline cellulose, silicon dioxide, croscarmellose sodium, crospovidone, hydroxypropylcellulose, methyl cellulose, povidone, magnesium aluminum silicate, starch or combinations thereof.

The surfactant that can be used in the pharmaceutical formulation of the present invention can be selected from, but not limited to, a group comprising docusate sodium, sorbitan esters, cetrimide and sodium lauryl sulfate. In the present invention, sodium lauryl sulfate is preferably used as the surfactant.

The pharmaceutical formulation of the present invention can comprise 20-700 mg cefpodoxime proxetil or its pharmaceutically acceptable salts, hydrates, solvates or combinations thereof in an equal amount.

The pharmaceutical formulation of the present invention can comprise 100-450 mg clavulanic acid or its pharmaceutically acceptable salts, hydrates, solvates or combinations thereof in an amount equivalent to that.

Clavulanic acid and its derivatives (e.g. potassium clavulanate) are extremely susceptible to moisture. To this respect, potassium clavulanate in the pharmaceutical formulation is preferably used with a humectant in the ratio of 1:1.

One or more of the substances comprising silica; colloidal silica, for instance colloidal silica anhydrous, for example Aerosil® 200, magnesium trisilicate, cellulose powder, Cabosil®, magnesium oxide, calcium silicate, Syloid®, starch, microcrystalline cellulose, talc can be used as the humectant.

In the pharmaceutical formulation of the present invention, potassium clavulanate is preferably used with microcrystalline cellulose and preferably in the ratio of 1:1.

The pharmaceutical formulation of the present invention can comprise 5-50% cefpodoxime proxetil in proportion to total weight of unit dose or pharmaceutically acceptable solvates, hydrates, enantiomers, racemates, organic salts, inorganic salts, polymorphs, crystalline and amorphous forms thereof.

The pharmaceutical formulation of the present invention can comprise 5-45% clavulanic acid in proportion to total weight of unit dose or pharmaceutically acceptable salts, hydrates, solvates or combinations thereof in an amount equivalent to that.

The pharmaceutical formulation of the present invention can comprise 5-50% cefpodoxime proxetil; 5-45% potassium clavulanate; 0,6-3% the first glidant; 0,6-3% the second glidant; 0,1-4% lubricant; 0,5-25% disintegrant and/or disintegrants; 0,5-15% diluent; 0,2-5% surfactant by weight and optionally coating agent in an amount of 1-4% by weight with respect to the core weight.

In another aspect, the present invention relates to processes for preparation of pharmaceutical formulations comprising pharmaceutically acceptable excipients in addition to cefpodoxime proxetil and clavulanic acid derivatives as the active agents and glidants.

According to this, the process of the present invention comprises the steps of granulating the active agents cefpodoxime proxetil and clavulanic acid derivatives by conventional wet and/or dry granulation methods; or powdering cefpodoxime proxetil, clavulanic acid derivatives and other excipients after mixing them by dry blending method and
- compressing the pharmaceutical formulation of the present invention in tablet form and optionally coating the tablets with a coating agent in the case that the product is developed in tablet form,
- filling the pharmaceutical formulation of the present invention in capsules in the case that the product is developed in tablet form,
- filling the pharmaceutical formulation of the present invention in packs in the case that the product is developed in sachet form.

Another aspect of the present invention is that the formulation prepared according to said invention is used in the treatment of diseases related with infections caused by gram negative and gram positive bacteria.

According to another aspect of the present invention, the pharmaceutical formulation prepared according to the present invention is used in the production of a medicament so as to be used in upper respiratory infections such as ear, nose, throat, otitis media, sinusitis, tonsillitis, pharyngitis; lower respiratory tract infections such as pyelonephritis, cystitis and urethritis; skin or soft tissue infections such as froncle, pyoderma, impetigo; in the treatment and prophylaxis of gonorrhea and lyme diseases.

The pharmaceutical formulation pertaining to the present invention can be prepared as described below, but not limited to the examples given.

**EXAMPLE** 1: Formulation and process for preparation of film tablet comprising cefpodoxime proxetil and sodium clavulanate

| | % of amount present in unit dose |
|---|---|
| **CORE** | |
| Cefpodoxime proxetil | 34% |
| Potassium clavulanate: Avicel | 36.5% |
| Disintegrant | 17% |
| Diluent | 7.5% |
| Surfactant | 1.5% |
| Lubricant | 1.5% |
| Talc | 1% |
| Silicon dioxide | 1% |

| **COATING** | |
|---|---|
| Coating agent | 3% |

According to this, a process for preparation of pharmaceutical formulations of the present invention is composed of the steps of mixing and compressing cefpodoxime proxetil, the disintegrant and the diluent and then sieving them; adding the surfactant, potassium clavulanate, microcrystalline cellulose, disintegrant, the glidant combination of talc and silicon dioxide and lubricant into the granules obtained and mixing them; and then compressing tablets of the mixture obtained and coating the tablets with coating material.

## Claims

1. A pharmaceutical formulation comprising cefpodoxime proxetil and clavulanic acid salts in combination **characterized in that** a combination of glidants consisting of a first glidant wherein said glidant consists of a mineral having Mohs' hardness value in the range of 1-5 and a second glidant wherein said glidant consists of a mineral having Mohs' hardness value in the range of 5.1-10 is used and the ratio of the first glidant to the second glidant is in the range of 2:1 to 1:3.

2. The pharmaceutical formulation according to claim 1 wherein the ratio of the first glidant to the second glidant is in the range of 1:1.

3. The pharmaceutical formulation according to claims 1-2, wherein potassium clavulanate is used.

4. The pharmaceutical formulation according to claims 1-3, wherein the first glidant consisting of a mineral having Mohs' hardness value in the range of 1-5 is selected from talc, tribasic calcium phosphate and calcium carbonate.

5. The pharmaceutical formulation according to claim 4, wherein talc is used as the first glidant.

6. The pharmaceutical formulation according to claims 1-5, wherein the second glidant consisting of a mineral having Mohs' hardness value in the range of 5.1-10 is selected from titanium dioxide, silicon dioxide (SiO₂) and magnesium silicate.

7. The pharmaceutical formulation according to claim 6, wherein silicon dioxide is used as the second glidant.

8. The pharmaceutical formulation according to claims 1-7, wherein said formulation is in conventional tablet, film coated tablet, sachet or capsule form.

9. The pharmaceutical formulation according to claims 1-8, wherein said formulation comprises one or more excipients comprising lubricant, disintegrant, diluent, surfactant and optionally coating agents in addition to cefpodoxime proxetil, potassium clavulanate and glidants.

10. The pharmaceutical formulation according to claim 9, wherein the amount of the diluent comprised in said formulation is in the range of 2-15% by weight.

11. The pharmaceutical formulation according to claim 10, wherein the amount of the diluent comprised in said formulation is in the range of 4-8% by weight.

12. The pharmaceutical formulation according to claims 1-11, wherein said formulation comprises 5-50% cefpodoxime proxetil; 5-45% potassium clavulanate; 0,6-3% the first glidant; 0,6-3% the second glidant; 0,1-4% lubricant; 0,5-25% disintegrant and/or disintegrants; 0,5-15% diluent; 0,2-5% surfactant by weight and optionally coating agent of 1-4% of the core weight.

13. A process for preparation of the pharmaceutical formulation according to claims 1-12, wherein said process comprises the steps of granulating the active agents cefpodoxime proxetil and clavulanic acid salts by conventional wet and/or dry granulation methods; or powdering cefpodoxime proxetil, clavulanic acid and other excipients after mixing them by dry blending method.

## Patentansprüche

1. Pharmazeutische Formulierung, umfassend Cefpodoximproxetil und Clavulansäuresalze in Kombination, **dadurch gekennzeichnet, dass** eine Kombination von Gleitmitteln, bestehend aus einem ersten Gleitmittel, wobei das genannte Gleitmittel, bestehend aus einem Mineral mit Mohs-Härtewert im Bereich von 1-5, und einem zweiten Gleitmittel, wobei das genannte Gleitmittel, bestehend aus einem Mineral mit Mohs-Härtewert im Bereich von 5.1-10, wird verwendet, und das Verhältnis des ersten Gleitmittels zum zweiten Gleitmittel liegt im Bereich von 2:1 bis 1:3.

2. Pharmazeutische Formulierung nach Anspruch 1, wobei das Verhältnis des ersten Gleitmittels zum zweiten Gleitmittel im Bereich von 1:1 liegt.

3. Pharmazeutische Formulierung nach den Ansprüchen 1-2, wobei Kaliumclavulanat verwendet wird.

4. Pharmazeutische Formulierung nach den Ansprüchen 1-3, wobei das erste Gleitmittel, bestehend aus einem Mineral mit Mohs-Härtewert im Bereich von 1-5, ist aus Talk, dreibasischem Calciumphosphat und Calciumcarbonat ausgewählt.

5. Pharmazeutische Formulierung nach Anspruch 4, wobei Talk als das erste Gleitmittel verwendet wird.

6. Pharmazeutische Formulierung nach den Ansprüchen 1-5, wobei das zweite Gleitmittel, bestehend aus einem Mineral mit Mohs-Härtewert im Bereich von 5.1-10, ist aus Titandioxid, Siliziumdioxid (SiO₂) und Magnesiumsilikat ausgewählt.

7. Pharmazeutische Formulierung nach Anspruch 6, wobei Siliziumdioxid als das zweite Gleitmittel verwendet wird.

8. Pharmazeutische Formulierung nach den Ansprüchen 1-7, wobei die genannte Formulierung in herkömmlicher Tabletten-, Filmtabletten-, Sachet- oder Kapselform ist.

9. Pharmazeutische Formulierung nach den Ansprüchen 1-8, wobei die genannte Formulierung, bestehend aus dem Schmiermittel, Sprengmittel, Verdünnungsmittel, Tensid und gegebenenfalls der Beschichtungsmittel zusätzlich zu Cefpodoximproxetil, Kaliumklavulanat und Gleitmitteln, einen oder mehrere Exzipienten umfasst.

10. Pharmazeutische Formulierung nach Anspruch 9, wobei die Menge des in der genannten Formulierung enthaltenen Verdünnungsmittels nach Gewicht im Bereich von 2-15% ist.

11. Pharmazeutische Formulierung nach Anspruch 10, wobei die Menge des in der genannten Formulierung enthaltenen Verdünnungsmittels nach Gewicht im Bereich von 4-8% ist.

12. Pharmazeutische Formulierung nach den Ansprüchen 1-11, wobei die genannte Formulierung nach Gewicht von 5-50% Cefpodoximproxetil; von 5-45% Kaliumclavulanat; von 0,6-3% des ersten Gleitmittels; von 0,6-3% des zweiten Gleitmittels; von 0,1-4% Schmiermittel; von 0,5-25% Sprengmittel und/oder Sprengmittel; von 0,5-15% Verdünnungsmittel; von 0,2-5% Tensid und gegebenenfalls Beschichtungsmittel von 1-4% des Kerngewichts umfasst.

13. Verfahren zur Herstellung der pharmazeutischen Formulierung nach den Ansprüchen 1-12, wobei das Verfahren die Schritte des Granulierens der Wirkstoffe Cefpodoximproxetil und Clavulansäuresalze durch herkömmliche Nass- und/oder Trockengranulations verfahren; oder des Pulverisierens von Cefpodoximproxetil, Clavulansäure und anderen Exzipienten nach deren Vermischen durch Trockenmischverfahren umfasst.

## Revendications

1. Formulation pharmaceutique comprenant du cefpodoxime proxétil et des sels d'acide clavulanique en combinaison **caractérisé en ce qu'**une combinaison des agents de glissement consistant en un premier agent de glissement dans laquelle ledit agent de glissement est constitué d'un minéral ayant une valeur de dureté de Mohs comprise entre 1-5 et d'un second agent de glissement dans laquelle ledit agent de glissement est constitué d'un minéral ayant une valeur de dureté de Mohs comprise entre 5.1-10 est utilisé et le rapport entre le premier agent de glissement et le deuxième agent de glissement est compris entre 2:1 à 1:3.

2. Formulation pharmaceutique selon la revendication 1, dans laquelle le rapport entre le premier agent de glissement et le deuxième agent de glissement est de l'ordre de 1:1.

3. Formulation pharmaceutique selon les revendications 1-2, dans laquelle le clavulanate de potassium est utilisé.

4. Formulation pharmaceutique selon les revendications 1-3, dans laquelle le premier agent de glissement, constitué d'un minéral ayant une valeur de dureté de Mohs comprise entre 1-5, est choisi parmi le talc, le phosphate de calcium tribasique et le carbonate de calcium.

5. Formulation pharmaceutique selon la revendication 4, dans laquelle le talc est utilisé comme premier agent de glissement.

6. Formulation pharmaceutique selon les revendications 1-5, dans laquelle le second agent de glissement, constitué d'un minéral ayant une valeur de dureté de Mohs comprise entre 5.1-10, est choisi parmi le dioxyde de titane, le dioxyde de silicium (SiO₂) et le silicate de magnésium.

7. Formulation pharmaceutique selon la revendication 6, dans laquelle le dioxyde de silicium est utilisé comme deuxième agent de glissement.

8. Formulation pharmaceutique selon les revendications 1-7, dans laquelle ladite formulation est sous forme de comprimé conventionnel, de comprimé pelliculé, de sachet ou de capsule.

9. Formulation pharmaceutique selon les revendications 1-8, dans laquelle ladite formulation comprend un ou plusieurs excipients comprenant un lubrifiant, un désintégrant, un diluant, un agent tensioactif et éventuellement des agents d'enrobage en plus du cefpodoxime proxétil, du clavulanate de potassium et des agents de glissement.

10. Formulation pharmaceutique selon la revendication 9, dans laquelle la quantité de diluant comprise dans ladite formulation se situe dans la plage de 2-15 % en poids.

11. Formulation pharmaceutique selon la revendication 10, dans laquelle la quantité de diluant comprise dans ladite formulation se situe dans la plage de 4-8 % en poids.

12. Formulation pharmaceutique selon les revendications 1-11, dans laquelle ladite formulation comprend 5-50% de cefpodoxime proxetil ; 5-45 % de clavulanate de potassium ; 0,6-3 % du premier agent de glissement ; 0,6-3 % du second agent de glissement ; 0,1-4 % de lubrifiant ; 0,5-25 % de désintégrant et/ou de désintégrants ; 0,5-15% de diluant ; 0,2-5% de tensioactif en poids et éventuellement un agent de revêtement de 1-4% du poids du noyau.

13. Procédé de préparation de la formulation pharmaceutique selon les revendications 1-12, dans lequel ledit procédé comprend les étapes de granulation des agents actifs cefpodoxime proxétil et sels d'acide clavulanique par des méthodes classiques de granulation par voie humide et/ou sèche ; ou de pulvérisation du cefpodoxime proxétil, de l'acide clavulanique et d'autres excipients après les avoir mélangés par la méthode du mélange à sec.
